# EUROPEAN PATENT APPLICATION

(11) **EP 4 778 465 A1**
(43) Date of publication of application: **22.07.2026**
(21) Application number: 26152253.6
(22) Date of filing: 16.01.2026
(51) Int. Cl.: A61B 6/00, A61B 6/58

(54) **RADIOGRAPHY SYSTEM**

(30) Priority: 21.01.2025 JP 2025008709
(71) Applicant: FUJIFILM Corporation, Tokyo 106-8620 (JP)
(72) Inventor: HORIUCHI, Hisatsugu, Tokyo, 106-8620 (JP); TANEICHI, Tatsuya, Tokyo, 106-8620 (JP); KOBAYASHI, Takeyasu, Tokyo, 106-8620 (JP); KITAMURA, Moeka, Tokyo, 106-8620 (JP)
(74) Representative: Dehns Germany Partnerschaft mbB

(57) **Abstract**

A radiography system includes a first position detection mechanism that includes an optical camera that is provided in the radiation generation apparatus and capable of imaging the electronic cassette, and detects a position of the electronic cassette based on a camera image captured by the optical camera, a second position detection mechanism that includes a generator that generates a non-optical signal and a sensor that detects the non-optical signal, in which one of the generator and the sensor is provided in the radiation generation apparatus and the other is provided in the electronic cassette, and detects a position of the electronic cassette based on the non-optical signal, and a processor that is configured to execute control of outputting a detection result of at least one of the first position detection mechanism or the second position detection mechanism.

## Description

### BACKGROUND OF THE INVENTION

### 1. Field of the Invention

The present disclosure relates to a radiography system.

### 2. Description of the Related Art

As an example of a radiography system including a radiation generation apparatus and a panel unit, a radiography system using X-rays is known. The radiography system includes a radiation generation apparatus including a radiation source that emits radiation as a radiation generation apparatus, and a panel unit including a radiation detector that detects the emitted radiation. As the panel unit, in addition to a stationary type, a portable type called an electronic cassette is known. The electronic cassette can be used for so-called free imaging in which imaging is performed without using an imaging table, unlike the stationary type panel unit including the imaging table. In the free imaging, the electronic cassette can be used in a variety of ways, such as imaging a patient (an example of a subject) lying on a bed in a patient's room, placing the electronic cassette against a joint of the patient's hand or foot to image the joint, and bringing the electronic cassette to a site of a disaster or the like for use.

In the radiography, relative alignment (so-called positioning) between the radiation source and the panel unit is required, but in a case in which the electronic cassette is used for the free imaging, although a degree of freedom of a position and a posture is high, it is difficult to ascertain a position of the electronic cassette with respect to the radiation source. Therefore, various techniques have been proposed for the alignment of the electronic cassette (JP2019-033826A and JP2008-237230A).

JP2019-033826A discloses a technique of detecting a position of the electronic cassette by using a position detection unit having a configuration independent of the radiation generation apparatus and the electronic cassette. In the position detection technique disclosed in JP2019-033826A, the position detection unit is disposed on a side of the electronic cassette, and detects the position of the electronic cassette from the side of the electronic cassette by, for example, an optical method. The position detection unit is imaged by the optical camera provided in the radiation source of the radiation generation apparatus, and the relative positional relationship between the radiation source and the electronic cassette is indirectly ascertained based on a position of the position detection unit appearing in an optical image obtained by the optical camera and a position of the electronic cassette detected by the position detection unit. Since the position detection unit disclosed in JP2019-033826A can detect the position of the electronic cassette from the side, the position of the electronic cassette can be detected even in a case in which the electronic cassette is hidden in a shadow of the patient as viewed from the radiation source side.

JP2008-237230A discloses a technique in which a plurality of wireless signal transmitters are provided on the electronic cassette and the position of the electronic cassette is detected based on a plurality of wireless signals. Since the position detection technique disclosed in JP2008-237230A uses a non-optical wireless signal, the position of the electronic cassette can be detected even in a case in which the electronic cassette is hidden in a shadow of the patient, as in JP2019-033826A.

### SUMMARY OF THE INVENTION

Both JP2019-033826A and JP2008-237230A are techniques that can detect the position in a case in which the electronic cassette is hidden, but the technique of JP2019-033826A uses the position detection unit having the configuration independent of the radiation generation apparatus and the electronic cassette, so that the configuration is complicated, and the portability of the electronic cassette may be impaired depending on the application. On the other hand, the technique of JP2008-237230A using the non-optical signal may have low detection accuracy. As described above, the position detection techniques in the related art of JP2019-033826A and JP2008-237230A have problems in terms of usability and there is room for improvement.

The technology of the present disclosure provides a radiography system capable of detecting a position of an electronic cassette with higher usability than that of the related art with a simpler configuration than that of the related art.

In order to achieve the above object, a radiography system according to the present disclosure includes a radiation generation apparatus, an electronic cassette, a first position detection mechanism that includes an optical camera that is provided in the radiation generation apparatus and capable of imaging the electronic cassette, and detects a position of the electronic cassette based on a camera image captured by the optical camera, a second position detection mechanism that includes a generator that generates a non-optical signal and a sensor that detects the non-optical signal, in which one of the generator and the sensor is provided in the radiation generation apparatus and the other is provided in the electronic cassette, and detects a position of the electronic cassette based on the non-optical signal, and a processor that is configured to execute control of outputting a detection result of at least one of the first position detection mechanism or the second position detection mechanism.

The radiography system may include two operation modes of a single mode in which the detection results of the first position detection mechanism and the second position detection mechanism are selectively used and a combined mode in which the detection results of both the first position detection mechanism and the second position detection mechanism are used.

The processor may be configured to display, on a display, a composite image in which a marker indicating the position of the electronic cassette and the subject are superimposed on each other as the detection result, and to change a display aspect of the marker in the composite image according to whether the detection result is acquired from the first position detection mechanism or the second position detection mechanism.

The processor may be configured to determine a way of using the first position detection mechanism and the second position detection mechanism based on a preset condition.

The processor may be configured to preferentially use the detection result of the first position detection mechanism.

The processor may be configured to determine whether or not the detection result of the first position detection mechanism is usable, and use the detection result of the second position detection mechanism in a case in which a determination is made that the detection result of the first position detection mechanism is unusable.

The processor may be configured to execute image recognition processing of performing image recognition of the electronic cassette from the camera image, and determine, based on a recognition result of the image recognition processing, whether or not the detection result of the first position detection mechanism is usable.

The recognition result may include at least one item among the number of corners of the electronic cassette that are recognized, an area of a region recognized as a part of the electronic cassette, a position of the region recognized as the part of the electronic cassette, or temporal stability of the recognition result, and the processor may be configured to evaluate a reliability of the detection result of the first position detection mechanism by using the at least one item as an evaluation index, and determine whether or not the detection result of the first position detection mechanism is usable.

The evaluation index may be the number of corners of the electronic cassette that are recognized, and the processor may be configured to use the detection result of the first position detection mechanism in a case in which one or more corners are recognized, and use the detection result of the second position detection mechanism in a case in which no corner is recognized.

The processor may be configured to display, on a display, a composite image in which a marker indicating the position of the electronic cassette and the subject are superimposed on each other as the detection result, generate the marker based on positions of three or more recognized corners in a case in which the number of the recognized corners is three or more, and generate the marker based on positions of one or two recognized corners and preset size information of the electronic cassette in a case in which the number of the recognized corners is one or two.

The processor may be configured to display, on a display, a composite image in which a marker indicating the position of the electronic cassette and the subject are superimposed on each other as the detection result, and change a display aspect of the marker in the composite image according to the reliability degree.

The processor may be configured to, in the combined mode, use the detection result of the second position detection mechanism to determine a reliability degree of the detection result of the first position detection mechanism.

The processor may be configured to display, on a display, a composite image in which a marker indicating the position of the electronic cassette and the subject are superimposed on each other as the detection result, and change a display aspect of the marker in the composite image according to the reliability degree.

The processor may be configured to detect a posture of the electronic cassette based on the camera image.

The non-optical signal may be a magnetic field or a radio wave, and the sensor may be a magnetic sensor or a wireless receiver.

The processor may be configured to stop transmission and reception of the non-optical signal in the second position detection mechanism at a timing of the radiography.

According to the above-described aspect, the technology of the present disclosure can detect the position of the electronic cassette with higher usability than that of the related art with a simpler configuration than that of the related art.

### BRIEF DESCRIPTION OF THE DRAWINGS

FIG. 1 is a diagram showing a manner of imaging using a radiography system.
FIG. 2 is a block diagram of the radiography system.
FIG. 3 is a diagram schematically showing processing of a first position detection mechanism.
FIG. 4 is a diagram schematically showing processing of a second position detection mechanism.
FIG. 5 is a diagram schematically showing processing of generating a composite image including a marker.
FIG. 6 is a diagram showing a positioning screen.
FIG. 7 is a diagram showing a state in which an entire electronic cassette is hidden in a patient.
FIG. 8 is a flowchart showing a processing procedure of the radiography.
FIG. 9 is a flowchart showing a processing procedure of cassette position detection processing.
FIG. 10 is a diagram schematically showing a change in a display aspect of the marker.
FIG. 11 is a flowchart showing an example in which a detection result of the first position detection mechanism is used preferentially.
FIG. 12 is a diagram showing an example in which the number of corners of the electronic cassette is used as an evaluation index.
FIG. 13 is a diagram showing an example in which an area of a region of the electronic cassette is used as an evaluation index.
FIG. 14 is a diagram showing an example in which a distance from a target region is used as an evaluation index.
FIG. 15 is a diagram showing an example in which temporal stability is used as an evaluation index.
FIG. 16 is a diagram showing an example of a combined mode.
FIG. 17 is a diagram showing the electronic cassette in a reference posture.
FIG. 18 is a diagram showing the electronic cassette rotated about a Z axis.
FIG. 19 is a diagram showing the electronic cassette rotated about an X axis.
FIG. 20 is a diagram showing a positioning screen with an added angle display.
FIG. 21 is a diagram showing a holder.

### DESCRIPTION OF THE PREFERRED EMBODIMENTS

### First Embodiment

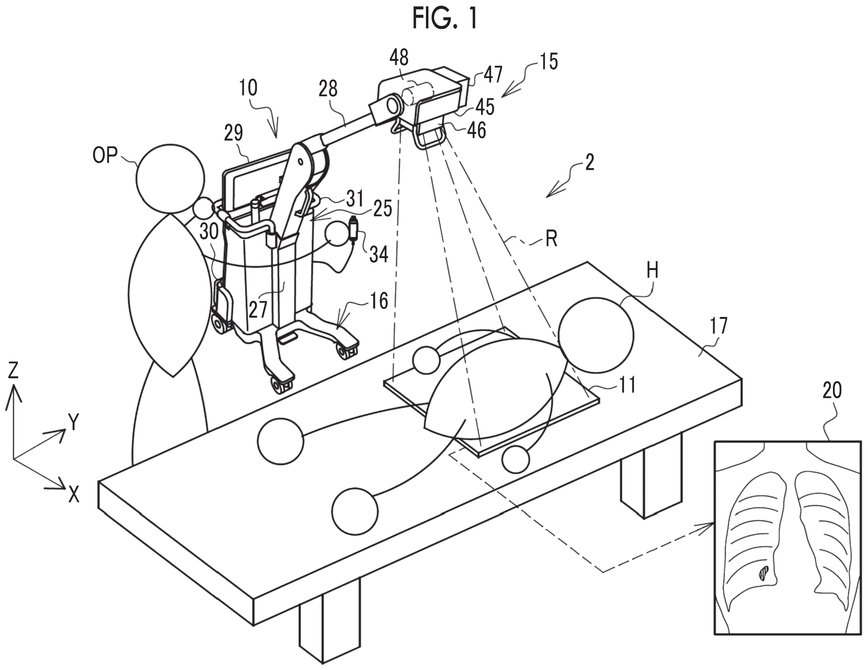

In FIG. 1, a radiography system 2 includes a radiation generation apparatus 10 and an electronic cassette 11. The radiation generation apparatus 10 is a movable type including a carriage unit 16 that can travel, and a main body portion 25 and a radiation source 15 are mounted on the carriage unit 16. The radiation source 15 generates radiation R. The radiation generation apparatus 10 is movable, for example, inside a hospital. The radiation generation apparatus 10 is used in so-called round imaging for imaging the patient H while visiting patient's rooms. Such a radiation generation apparatus 10 is also referred to as a mobile X-ray unit. Alternatively, the radiation generation apparatus 10 is also used in imaging in an emergency room. In addition, the radiation generation apparatus 10 can also be brought into a surgery room and used during the surgery. The radiation generation apparatus 10 can also be carried in an outdoor disaster site and used in emergency. The radiation generation apparatus 10 is an example of a "radiation generation apparatus" according to the technology of the present disclosure. The patient H is an example of a "subject" according to the technology of the present disclosure.

As is well known, the electronic cassette 11 is a radiation image detection device in which a sensor panel constituting a radiation detector that detects radiation is incorporated in a portable housing. The electronic cassette 11 is driven by, for example, a battery, and can also perform wireless communication with the radiation generation apparatus 10. As is also well known, the sensor panel has a configuration in which a plurality of pixels that are sensitive to the radiation R or visible light converted from the radiation R to generate signal charge are arranged in a matrix. The electronic cassette 11 is placed, for example, below the patient H, detects the radiation R emitted from the radiation source 15 and transmitted through the patient H, and outputs a radiographic image 20 of the patient H. The electronic cassette 11 has a plurality of types in terms of vertical and horizontal size, including 17 inches (431.8 mm) × 17 inches (431.8 mm), 17 inches (431.8 mm) × 14 inches (355.6 mm), 12 inches (304.8 mm) × 10 inches (254 mm), and the like. Since the electronic cassette 11 is portable type, free imaging in which the electronic cassette 11 is used without being fixed to an imaging table as shown in FIG. 1 is possible.

The radiation generation apparatus 10 includes the main body portion 25, a column portion 27, an arm portion 28, and the like. The main body portion 25 includes an operation panel 29 and a handle 31. The handle 31 is used in a case in which the radiation generation apparatus 10 is moved by causing the carriage unit 16 to travel.

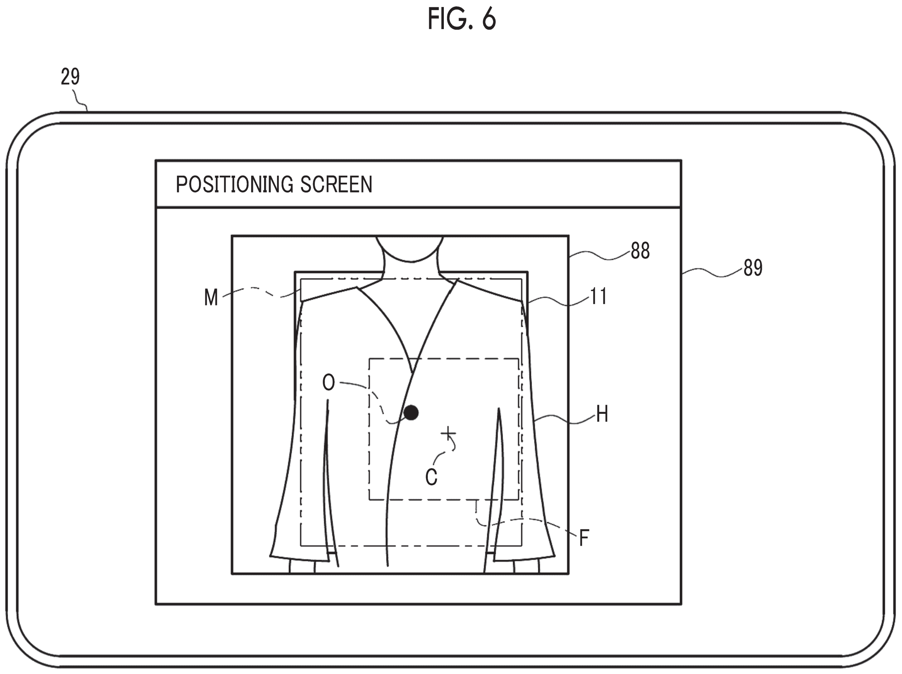

In addition, a housing portion 30 that houses the electronic cassette 11 is provided on a rear surface of the main body portion 25. The operation panel 29 is configured as, for example, a touch panel display (hereinafter, simply referred to as a display), and functions as a display that displays information in addition to an operation function. The operation panel 29 displays the radiation image 20 and the like. The operation panel 29 is operated by an operator OP such as a radiological technologist. The operator OP is an example of a user of the radiography system 2. An irradiation condition of the radiation R is set through the operation panel 29. In addition, the operation panel 29 is also used to check the captured radiation image 20. Further, as will be described below, a positioning screen 89 (see FIG. 6) is displayed on the operation panel 29. The positioning screen 89 is a screen that displays positioning support information for supporting the operator OP to perform positioning, which is relative alignment between the radiation source 15 and the electronic cassette 11, in a case of performing the positioning.

The irradiation switch 34 is a switch that is provided to allow the operator OP to give an instruction to start irradiation of radiation. An extension cable is connected to the irradiation switch 34, and can be detached from the main body portion 25 for use. The irradiation switch 34 is, for example, a two-stage push switch. The irradiation switch 34 generates a warm-up instruction signal when being pushed to the first stage (half-pushed), and generates an irradiation start instruction signal when being pushed to the second stage (fully pushed).

The column portion 27 has, for example, a prismatic columnar shape and is provided upright at the center of the carriage unit 16. The arm portion 28 has a base end that is attached to the column portion 27, and a distal end that is a free end on an opposite side to the base end and to which the radiation source 15 is attached.

The column portion 27 has a first column and a second column that is consecutively provided upward at a predetermined angle from the first column. The first column is provided on an upper surface of the carriage unit 16, and the second column can rotate with respect to the first column with a vertical axis as a rotation axis. The arm portion 28 can be bent with respect to the second column or can extend in a direction along the second column. In addition, the radiation source 15 can swing front and back with respect to the arm portion 28. By the displacement of the arm portion 28 and the displacement of the radiation source 15 with respect to the arm portion 28, a height of the radiation source 15 and an irradiation direction can be adjusted.

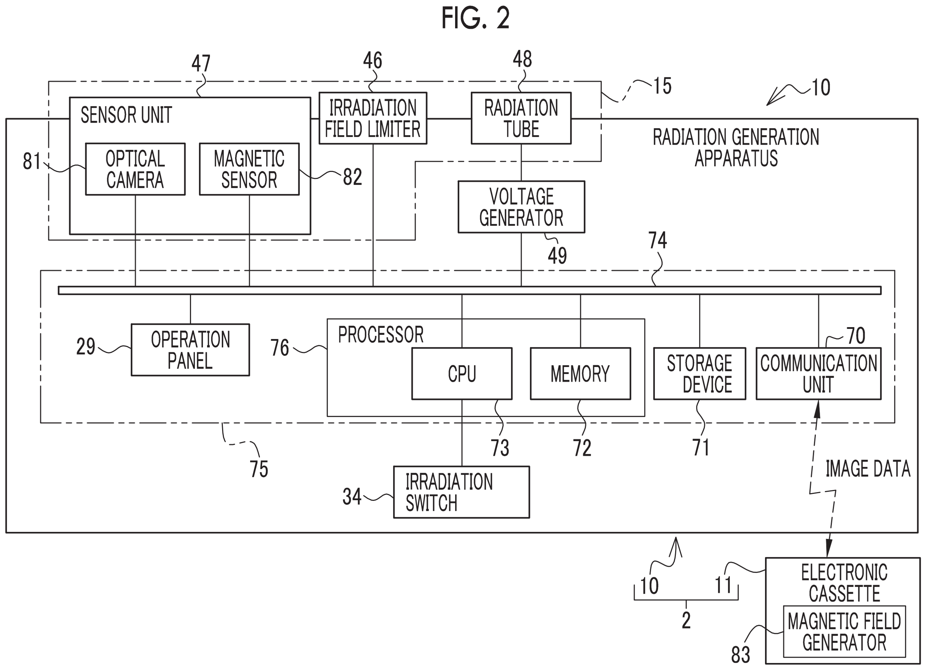

The radiation source 15 is configured of a radiation tube 48 and an irradiation field limiter 46. The radiation tube 48 generates, for example, X-rays as radiation R. The radiation tube 48 is provided with a filament, a target, a grid electrode, and the like (all are not shown). A tube voltage from a voltage generator 49 (see FIG.2) incorporated in the main body portion 25 is applied between the filament as a cathode and the target as an anode. The filament emits thermoelectrons toward the target in response to the applied tube voltage, and the target emits the radiation R by collision of the thermoelectrons from the filament. The grid electrode is disposed between the filament and the target, and changes a flow rate of the thermoelectrons from the filament toward the target in response to the voltage applied from the voltage generator 49. The flow rate of the thermal electrons from the filament toward the target is referred to as a tube current. The tube voltage and the tube current are set as irradiation conditions along with an irradiation time.

The irradiation field limiter 46 limits an irradiation field of radiation R generated from the radiation tube 48. For example, the irradiation field limiter 46 has a configuration in which four shield plates formed of lead or the like shielding radiation R are disposed on respective sides of a quadrangle, and an emission opening of the quadrangle transmitting radiation is formed in a center portion. The irradiation field limiter 46 changes the positions of the shield plates to change the size of the emission opening, and accordingly, changes the irradiation field of radiation R. The irradiation field limiter 46 is also called a collimator or the like.

In addition, a sensor unit 47 is provided in the radiation source 15. The sensor unit 47 constitutes a position detection mechanism that detects a position of the electronic cassette 11. As will be described below, the radiation generation apparatus 10 generates a composite image 88 of FIG. 6 as the positioning support information by using a detection result of the position detection mechanism including the sensor unit 47, and provides the generated composite image 88 to the operator OP through the positioning screen 89 (see FIG. 6).

The radiation generation apparatus 10 is equipped with a battery, can be driven by being supplied with power from the battery, and can also be supplied with power from a commercial power supply using a power supply cord.

In FIG. 2, the radiation generation apparatus 10 has a communication unit 70, a storage device 71, a memory 72, a central processing unit (CPU) 73, and the like. The communication unit 70, the storage device 71, the memory 72, the CPU 73, and the like are connected to one another through a busline 74. The operation panel 29, the irradiation field limiter 46, the voltage generator 49, and the sensor unit 47 are also connected to the busline 74. The communication unit 70, the storage device 71, the memory 72, the CPU 73, the busline 74, and the operation panel 29 constitute a console 75. The CPU 73 and the memory 72 constitute a processor 76. The processor 76 is an example of a "processor" according to the technology of the present disclosure.

The communication unit 70 includes a wireless communication interface that performs wireless communication with the electronic cassette 11. The electronic cassette 11 has a wireless communication function, and transmits image data of the captured radiation image 20 to the console 75 through the communication unit 70. The communication unit 70 includes a network interface that performs wireless communication with an external device other than the electronic cassette 11 through a network. Examples of the external device include a radiology information system (RIS) that manages information, such as an imaging order and picture archiving and communication systems (PACS). Examples of the network include a wide area network (WAN), such as the Internet or a public communication network.

The storage device 71 is, for example, a hard disk drive or a solid state drive, and stores various programs and various kinds of data associated with various programs. The memory 72 is a work memory on which the CPU 73 executes processing. The CPU 73 reads a program stored in the storage device 71 to the memory 72 and executes processing compliant with the read program. As a result, the processor 76 including the CPU 73 integrally controls operations of each unit of the radiation generation apparatus 10. The control program stored in the storage device 71 is an example of an operation program for causing the processor 76 to function as the radiography system 2. In addition, the storage device 71 stores various types of data for generating the positioning support information.

The sensor unit 47 includes an optical camera 81 and a magnetic sensor 82. The sensor unit 47 constitutes a position detection mechanism in cooperation with the processor 76. The position detection mechanism includes two types of mechanisms having different detection methods of a first position detection mechanism and a second position detection mechanism. The optical camera 81 constitutes the first position detection mechanism, and the magnetic sensor 82 constitutes the second position detection mechanism.

The optical camera 81 is a visible light camera having an image sensor that is sensitive to visible light, and is an example of an "optical camera" according to the technology of the present disclosure. The image sensor is a complementary metal oxide semiconductor (CMOS) image sensor, a charge coupled device (CCD) image sensor, or the like. The optical camera 81 is provided in the radiation source 15, and can image the electronic cassette 11.

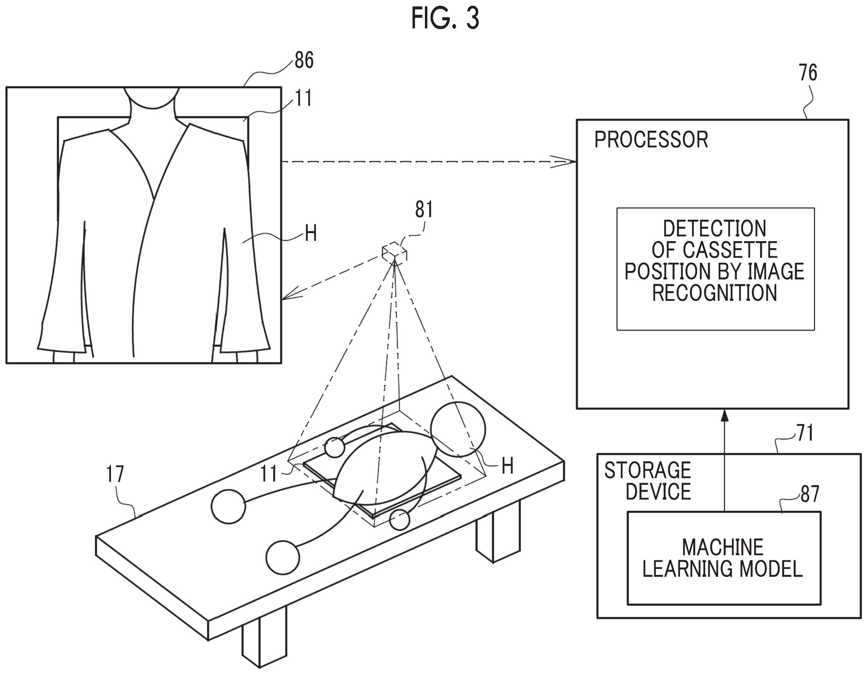

As shown in FIG. 3, the first position detection mechanism includes the optical camera 81, and detects the position of the electronic cassette 11 based on a camera image 86 captured by the optical camera 81. That is, the first position detection mechanism is a position detection mechanism that optically detects the position, and is an example of a "first position detection mechanism" according to the technology of the present disclosure. Since the optical camera 81 is provided in the radiation source 15, in a case in which the detection surface of the electronic cassette 11 disposed at an imaging site of the patient H and the radiation source 15 face each other, the optical camera 81 also faces the patient H and the electronic cassette 11. Therefore, in a case in which imaging is performed using the optical camera 81 in this state, the camera image 86 in a state in which the electronic cassette 11 is disposed behind the patient H can be acquired.

The camera image 86 is transmitted to the processor 76. The processor 76 performs image recognition based on the camera image 86 to detect the position of the electronic cassette 11 (hereinafter, also referred to as a cassette position). The image recognition is performed by, for example, the processor 76 using a machine learning model 87. The machine learning model 87 is stored in the storage device 71. The machine learning model 87 is, for example, an image recognition model that executes semantic segmentation capable of recognizing an object in an image, and recognizes a region of the electronic cassette 11 from the camera image 86. Coordinates and an area of the region are derived based on the recognized region. In addition, the items to be recognized include the position and the number of corners of the electronic cassette 11 in the region recognized as the electronic cassette 11. Examples of the image recognition model that executes the semantic segmentation include a convolutional neural network. Of course, the image recognition may not use the machine learning model 87, and may use a rule-based image processing method such as pattern matching.

For example, in a case in which three or more corners of the electronic cassette 11 can be recognized from the camera image 86, the position of the center of the detection surface of the electronic cassette 11 can be obtained by calculation from three corners. In addition, in a case in which the position of the corner and the position of the center of the detection surface are known, the contour of the electronic cassette 11 can also be derived. As described above, the first position detection mechanism detects the position of the center of the detection surface of the electronic cassette 11, the position of the corner, the contour of the electronic cassette 11, and the like as the cassette position by performing the image recognition. The cassette position detected in this way is the detection result of the first position detection mechanism.

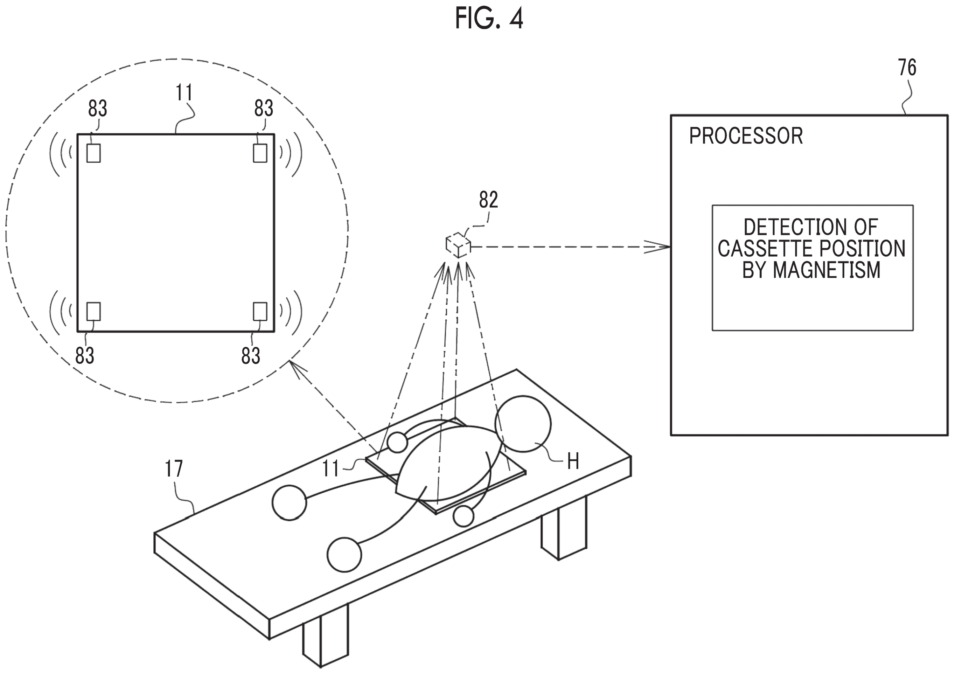

As shown in FIG. 4, the second position detection mechanism includes the magnetic sensor 82 and a magnetic field generator 83, and detects the position of the electronic cassette 11 by magnetism. That is, the second position detection mechanism is a position detection mechanism that detects the position in a non-optical manner, and is an example of a "second position detection mechanism" according to the technology of the present disclosure. The magnetic field generator 83 generates a magnetic field, and the magnetic field generator 83 is provided in the electronic cassette 11. The magnetic sensor 82 is a sensor that detects the magnetic field generated by the magnetic field generator 83.

The magnetic field generator 83 is disposed at each of the four corners of the electronic cassette 11. For example, the four magnetic field generators 83 are selectively operated to generate the magnetic field in order. The magnetic sensor 82 detects the magnetic fields generated by the four magnetic field generators 83 in order, and outputs a magnetic field detection signal representing a strength of each magnetic field to the processor 76. The strength of the magnetic field decreases as the distance increases. The processor 76 measures the distance between the four corners of the electronic cassette 11 based on the strength of each magnetic field generated by the four magnetic field generators 83. For example, in a case in which the distance from the magnetic sensor 82 provided in the radiation source 15 is known for three or more corners, the position of the center of the detection surface of the electronic cassette 11 can be detected as the cassette position from the positional relationship between the three or more corners. In addition, in a case in which the position of the center of the detection surface is known, the contour of the electronic cassette 11 can also be derived by using the preset size information of the electronic cassette 11. As described above, the second position detection mechanism detects the position of the center of the detection surface, the position of the corner of the electronic cassette 11, and the like as the cassette position by the magnetism. The cassette position detected in this way is the detection result of the second position detection mechanism.

The position at which the magnetic field generator 83 is disposed and the number thereof are merely examples, and are not limited thereto. For example, the number of the magnetic field generators 83 may be three, or conversely, may be five or more. In a case in which four or more magnetic field generators 83 are provided, for example, three magnetic field generators 83 having a higher magnetic field strength are selected from the four or more magnetic field generators 83, and the selected magnetic field generators 83 may be used for detecting the cassette position. In addition, the position at which the magnetic field generator 83 is provided may also be, for example, disposed closer to the center than the corner. Even at a position other than the corner, in a case in which the positional relationship between the three magnetic field generators 83 is known, the position of the center of the detection surface can be detected from the positional relationship.

The magnetic field is an example of a "non-optical signal" according to the technology of the present disclosure, the magnetic sensor 82 is an example of a "sensor that detects a non-optical signal", and the magnetic field generator 83 is an example of a "generator that generates a non-optical signal".

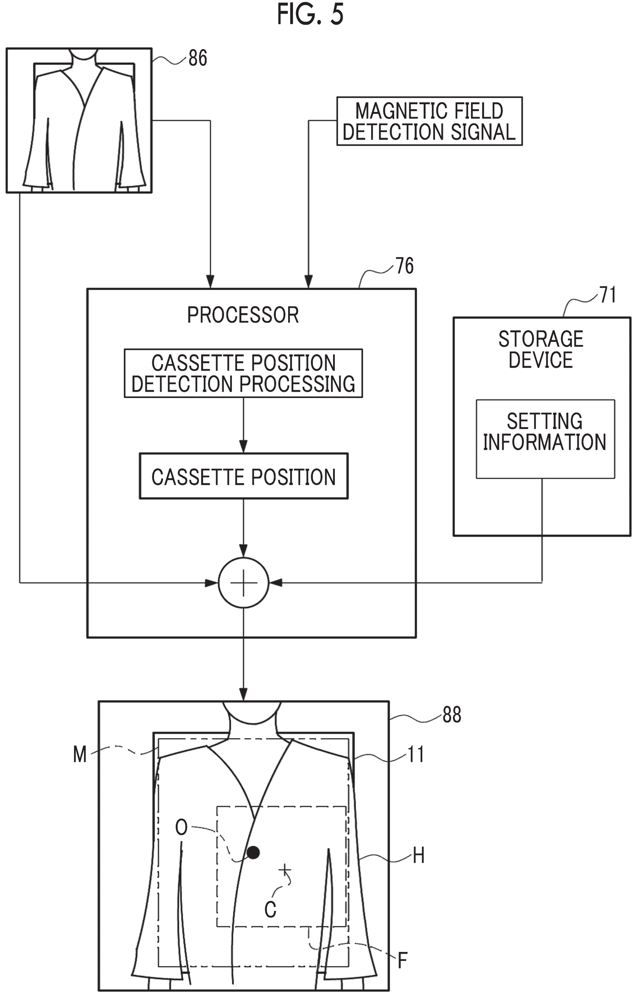

As shown in FIG. 5, the processor 76 executes control of outputting the detection result of at least one of the first position detection mechanism or the second position detection mechanism. The processor 76 is an example of a "processor" according to the technology of the present disclosure. The processor 76 executes at least one of the cassette position detection processing based on the camera image 86 shown in FIG. 3 or the cassette position detection processing based on the magnetic field detection signal shown in FIG. 4 as the cassette position detection processing, to detect the cassette position. Then, the processor 76 generates, as the detection result, for example, the composite image 88 in which a marker M indicating the cassette position and the patient H are superimposed on each other. The composite image 88 is obtained by superimposing the marker M on the camera image 86 in which the patient H and the electronic cassette 11 are imaged. The marker M is, for example, a marker indicating the contour of the electronic cassette 11 and a center O. In addition, as an example, a marker F indicating an irradiation field of the radiation source 15 and a marker indicating a center C of the irradiation field are also superimposed on the composite image 88.

The setting information of the storage device 71 records a focal length, an angle of view, size information of the electronic cassette 11, and a positional relationship between the optical camera 81 and the irradiation field limiter 46 of the optical camera 81. For example, in a case in which the focal length, the angle of view, and the positional relationship between the optical camera 81 and the irradiation field limiter 46 of the optical camera 81 are known, the position of the irradiation field in the camera image 86 can be derived. The processor 76 uses the setting information as necessary to generate the composite image 88 including the marker M and the marker F.

The camera image 86 or the magnetic field detection signal is acquired at, for example, a constant interval, and the processor 76 updates the composite image 88 based on the latest camera image 86 and magnetic field detection signal. Therefore, in a case in which the electronic cassette 11 is moved, the position of the marker M in the composite image 88 is also updated, and the latest position of the electronic cassette 11 is displayed in the composite image 88.

Then, as shown in FIG. 6, the processor 76 executes control of displaying the generated composite image 88 on the operation panel 29. As shown in FIG. 6, the composite image 88 is displayed on the positioning screen 89 and is checked by the operator OP.

The operator OP can manually select which of the first position detection mechanism or the second position detection mechanism is used, for example.

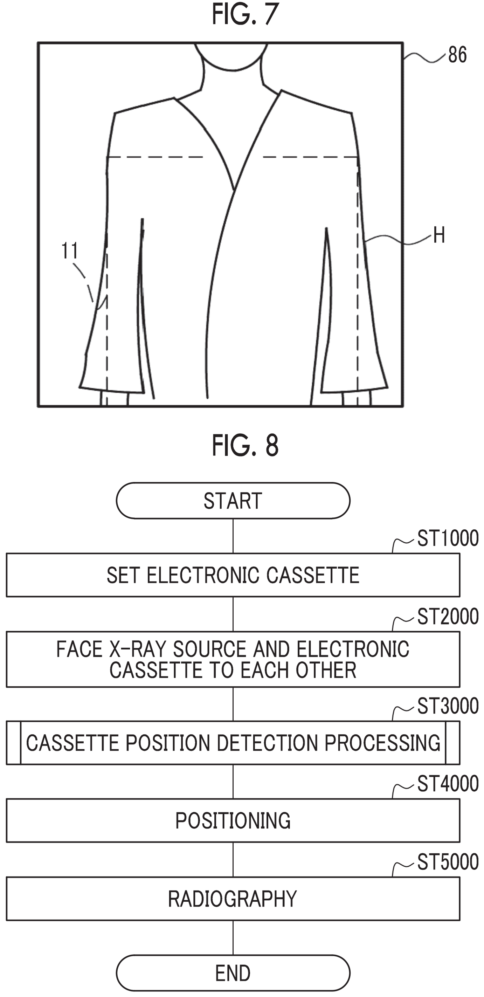

For example, as shown in FIG. 7, the entire electronic cassette 11 may be hidden in the shadow of the patient H. In this case, since the cassette position cannot be detected from the camera image 86, the detection result of the first position detection mechanism cannot be used. In this case, the second position detection mechanism is used to detect the position of the electronic cassette by the magnetism. As described above, in a case in which the entire electronic cassette 11 is hidden in the shadow of the patient H, the second position detection mechanism is required. However, in terms of reliability of the detection result, a method of the image recognition using the first position detection mechanism is more advantageous in many cases. Therefore, in a case in which the electronic cassette 11 appears in the camera image 86, it is preferable to select the first position detection mechanism as much as possible.

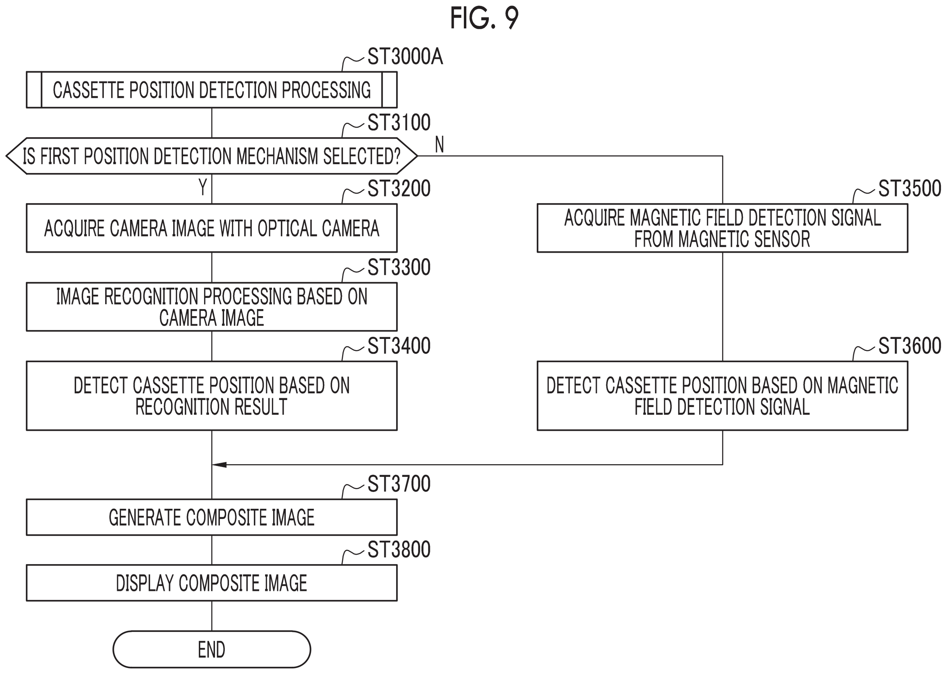

Hereinafter, the operation of the above-described configuration will be described with reference to the flowcharts shown in FIGS. 8 and 9. In a case of imaging the patient H as shown in FIG. 1, in step ST1000 shown in FIG. 8, the operator OP first sets the electronic cassette 11 behind a site of the patient H to be imaged. In a case in which the patient H is lying on the examination table 17, the electronic cassette 11 is inserted between the top plate of the examination table 17 and the patient H. Then, in step ST2000, the operator OP faces the radiation source 15 and the electronic cassette 11 to each other according to the position of the electronic cassette 11 to perform rough alignment.

Then, in step ST3000, the operator OP causes the radiation generation apparatus 10 to execute the cassette position detection processing. By executing the cassette position detection processing, the positioning screen 89 including the composite image 88 is displayed on the operation panel 29.

In step ST4000, the operator OP checks the cassette position by the marker M of the composite image 88, and checks the irradiation field of the radiation source 15 by the marker F. The operator OP performs the positioning of the radiation source 15 and the electronic cassette 11 while checking these positions.

After the positioning is completed, in step ST5000, the operator OP operates the radiography system 2 to perform the radiography. As a result, the radiation image 20 in which the positioning is appropriately performed is acquired.

FIG. 9 shows step ST3000A that is an example of the cassette position detection processing of step ST3000 shown in FIG. 8. Step ST3000A shown in FIG. 9 is an example in which the operator OP manually selects which of the first position detection mechanism or the second position detection mechanism is used. The selection of the position detection mechanism is performed, for example, through the operation panel 29, and the selection information is recorded in the storage device 71 as the setting information.

First, in step ST3100, the processor 76 checks the selection information of the position detection mechanism. In a case in which the first position detection mechanism is selected (Y in step ST3100), the processing proceeds to step ST3200, and in a case in which the first position detection mechanism is not selected (N in step ST3100), the processing proceeds to step ST3500.

In step ST3200, the processor 76 operates the optical camera 81 to acquire the camera image 86. Next, in step ST3300, the processor 76 executes image recognition processing based on the camera image 86. As described in FIG. 3, the image recognition processing is performed by using the machine learning model 87. Next, in step ST3400, the processor 76 detects the cassette position based on the recognition result.

Then, in step ST3700, as shown in FIG. 5, the processor 76 inserts the detected cassette position as the marker M into the camera image 86 to generate the composite image 88. In step ST3800, as shown in FIG. 6, the processor 76 displays the generated composite image 88 on the operation panel 29.

In addition, in step ST3100, in a case in which the second position detection mechanism is selected instead of the first position detection mechanism, the processor 76 operates the second position detection mechanism. In step ST3500, the processor 76 acquires the magnetic field detection signal from the magnetic sensor 82. Then, in step ST3600, as shown in FIG. 4, the cassette position is detected based on the magnetic field detection signal. After step ST3600 is ended, the processing proceeds to step ST3700. The processing of step ST3700 and step ST3800 is as described above.

As described above, a radiography system 2 according to the technology of the present disclosure for imaging a patient H (an example of a subject) includes a radiation generation apparatus 10, an electronic cassette 11, a first position detection mechanism that includes an optical camera 81 that is provided in the radiation generation apparatus 10 and capable of imaging the electronic cassette 11, and detects a position of the electronic cassette 11 based on a camera image 86 captured by the optical camera 81, a second position detection mechanism that includes a magnetic field generator 83 (an example of a generator) that generates a magnetic field (an example of a non-optical signal) and a magnetic sensor 82 (an example of a sensor) that detects the magnetic field, in which one of the magnetic field generator 83 and the magnetic sensor 82 is provided in the radiation generation apparatus 10 and the other is provided in the electronic cassette 11, and detects a position of the electronic cassette 11 by the magnetism (that is, based on the magnetic field), and a processor 76 that executes control of outputting a detection result of at least one of the first position detection mechanism or the second position detection mechanism.

As a result, the position of the electronic cassette **11** can be detected with higher usability than that of the related art with a simpler configuration than that of the related art. That is, since the position detection unit having a configuration independent of the radiation generation apparatus 10 and the electronic cassette **11** is not used, the configuration is simple. **In** addition, since position detection mechanisms with different detection methods, each having respective advantages, can be selectively used, the usability is improved. For example, the first position detection mechanism that performs optical detection has higher detection accuracy than the second position detection mechanism that detects the cassette position based on the non-optical signal such as the magnetic field. On the other hand, the second position detection mechanism may detect the cassette position even in a case in which the entire electronic cassette 11 is hidden in the shadow of the patient H. As described above, since the first position detection mechanism and the second position detection mechanism have respective advantages, the usability is high in that it is possible to use the first position detection mechanism and the second position detection mechanism according to the situation.

In addition, in the above-described embodiment, since the optical camera 81 is provided in the radiation source 15, the electronic cassette 11 can be imaged from a front surface on which the radiation source 15 is disposed. Therefore, higher detection accuracy can be expected as compared with a case in which the optical camera 81 is provided at another location of the radiation generation apparatus 10.

In addition, in the above-described embodiment, the non-optical signal is the magnetic field, and the magnetic field is detected by using the magnetic sensor 82. A large number of products of the magnetic field generator 83 and the magnetic sensor 82 are easily available. Therefore, it can be realized at a relatively low cost. In the above example, the example has been described in which the magnetic field generator 83 is provided in the electronic cassette 11 and the magnetic sensor 82 is provided in the radiation generation apparatus 10, but the magnetic sensor 82 may be provided in the electronic cassette 11 and the magnetic field generator 83 may be provided in the radiation generation apparatus 10. However, in this case, a configuration for transmitting the magnetic field detection signal from the electronic cassette 11 to the radiation generation apparatus 10 is required. Therefore, it is preferable to provide the magnetic sensor 82 in the radiation generation apparatus 10.

The non-optical signal may be a radio wave instead of the magnetic field. Since the radio wave also attenuates according to the distance as in the magnetic field, the distance information can be acquired. In a case of the radio wave, a wireless transmitter is used as the generator, and a wireless receiver is used as the sensor. Since there are many general-purpose products of the wireless transceiver, it is cost-effective as compared with the magnetic sensor 82. As the wireless receiver, a radio frequency identification (RFID) tag may be used. In addition, as the wireless communication, ultra-wideband (UWB) radio waves as the radio waves may be used for ultra-wideband wireless communication. In addition, in a case of using the magnetic field, distance measurement using an alternating magnetic field may be used.

### Modification Example of First Embodiment

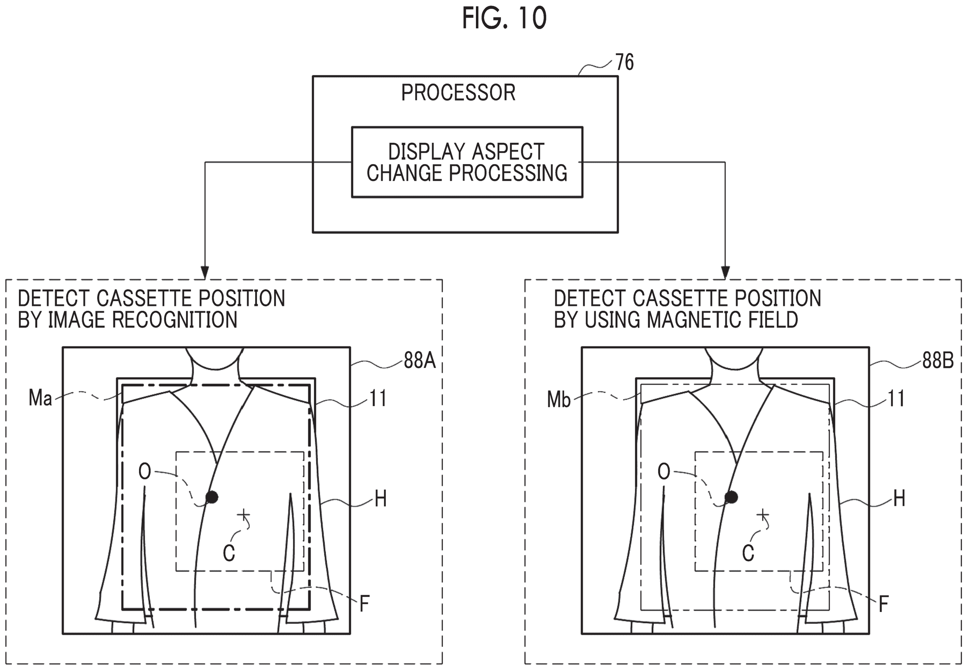

In addition, as shown in FIG. 10, the processor 76 may display, on the operation panel 29 (an example of a display), the composite image 88 in which the marker M indicating the position of the electronic cassette 11 and the patient H (an example of a subject) are superimposed on each other as the detection result, and may change a display aspect of the marker M in the composite image 88 according to whether the detection result is acquired from the first position detection mechanism or the second position detection mechanism. For example, in a case in which the reliability degree of the detection result is different between the first position detection mechanism and the second position detection mechanism, it may be convenient for the operator OP who is the user to ascertain the reliability degree of the detection result.

That is, in a case in which the cassette position is detected by the image recognition using the first position detection mechanism, the processor 76 generates a composite image 88A including a marker Ma, and in a case in which the cassette position is detected by the magnetism using the second position detection mechanism, the processor 76 generates a composite image 88B including a marker Mb. As described above, the detection accuracy of the detection result of the first position detection mechanism is higher than the detection accuracy of the detection result of the second position detection mechanism. By changing the display aspect of the marker Ma of the composite image 88A that is the detection result of the first position detection mechanism and the marker Mb of the composite image 88B that is the detection result of the second position detection mechanism, the user can clearly recognize which detection result is used. As a result, the user can ascertain the reliability degree of the detection result at a glance, so that the user can perform the positioning with attention in a case in which the reliability degree is low.

The display aspect may be any aspect as long as the marker Ma and the marker Mb can be identified. For example, the thickness of the line of the marker Ma, the color or brightness of the line, the line type, and the like are changed. In addition, either the marker Ma or the marker Mb may be made conspicuous, but for example, it is preferable to make the marker Ma having high reliability degree conspicuous. This is because it is considered that it is easier to intuitively ascertain that the reliability degree is high in a case in which the marker is conspicuous. FIG. 10 is an example in which the display aspect is changed by changing the thickness of the line and the line type. In FIG. 10, the thickness of the line of the marker Ma is larger than the thickness of the line of the marker Mb, and the marker Ma is indicated by a single dot chain line and the marker Mb is indicated by a two-dot chain line.

### Second Embodiment

In the first embodiment, the operator manually selects which of the first position detection mechanism or the second position detection mechanism is used, but in the second embodiment, the processor 76 determines a way of using the first position detection mechanism and the second position detection mechanism based on a preset condition. For example, since the processor 76 automatically determines the way of using the first position detection mechanism and the second position detection mechanism according to the situation, it may be more convenient than the case of manual selection.

The configuration of the radiography system 2 of the second embodiment is substantially the same as that of the first embodiment. Hereinafter, the same configurations will not be described, and the differences will be mainly described.

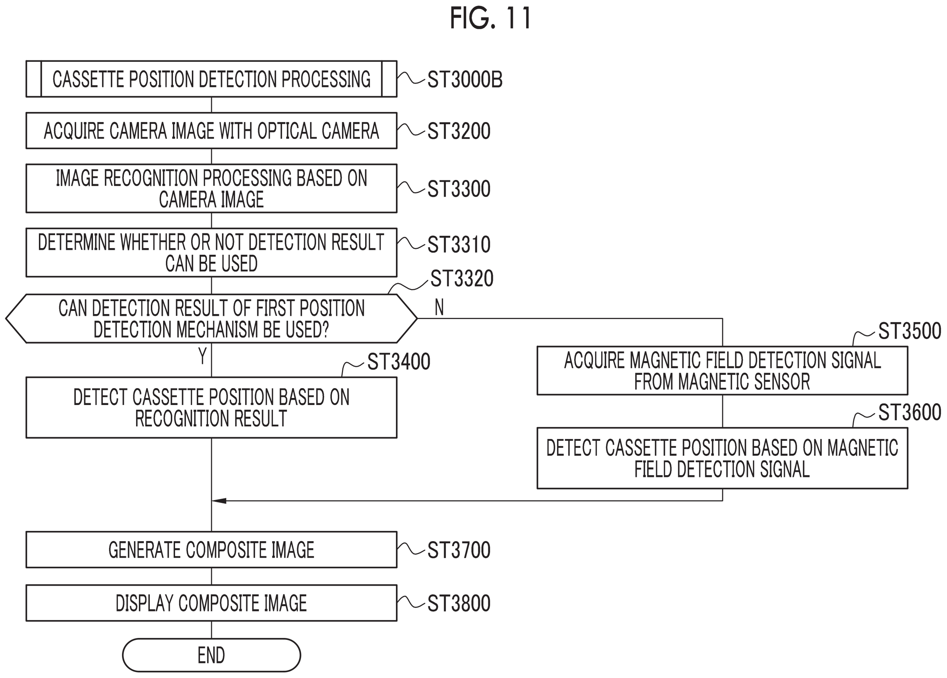

FIG. 11 is a flowchart showing a processing procedure of the cassette position detection processing in the second embodiment. Step ST3000B shown in FIG. 11 is an example of step ST3000 shown in FIG. 8 of the first embodiment. In the first embodiment, step ST3000A shown in FIG. 9 is adopted, but in the second embodiment, step ST3000B is adopted as step ST3000.

In step ST3000B shown in FIG. 11, the same step numbers are assigned to steps having the same contents as the steps included in step ST3000A shown in FIG. 9. The processing having the same step number will be omitted from the description in principle. In step ST3000B, the processor 76 first acquires the camera image 86 in step ST3200, and executes the image recognition processing based on the camera image 86 of step ST3300. In the second embodiment, in step ST3310, the processor 76 determines whether or not the detection result of the first position detection mechanism can be used based on the recognition result of the image recognition processing. This point is the main difference from the first embodiment in which the position detection mechanism is manually selected.

Then, the processor 76 proceeds to step ST3320, and in a case in which a determination is made that the detection result of the first position detection mechanism can be used (Y in step ST3320), the processor 76 proceeds to step ST3400. Subsequently, step ST3700 and step ST3800 are executed in order. The procedure of this processing is the same as that of the first embodiment. In addition, in step ST3320, in a case in which a determination is made that the detection result of the first position detection mechanism cannot be used (N in step ST3320), the processor 76 proceeds to step ST3500. Then, step ST3600, step ST3700, and step ST3800 are executed in order. The procedure of this processing is also the same as that of the first embodiment. As described above, the processor 76 preferentially uses the detection result of the first position detection mechanism. Specifically, the processor 76 determines whether or not the detection result of the first position detection mechanism can be used, and uses the detection result of the second position detection mechanism in a case in which a determination is made that the detection result of the first position detection mechanism cannot be used.

By preferentially using the detection result of the first position detection mechanism, the following advantages are obtained. That is, since the detection accuracy of the first position detection mechanism is often higher, the frequency at which the position can be accurately detected is increased by preferentially using the detection result of the first position detection mechanism. In addition, the first position detection mechanism using the optical camera 81 may be advantageous in terms of power consumption as compared with a case of using the second position detection mechanism using the non-optical signal such as the magnetic field. By preferentially using the first position detection mechanism, it is advantageous in terms of the detection accuracy, the power consumption, and the like.

In addition, by using the detection result of the second position detection mechanism in a case in which the detection result of the first position detection mechanism cannot be used, the following advantages are obtained. That is, the detection result of the first position detection mechanism having high reliability degree can be used as much as possible. In addition, in a case in which the composite image 88 in which the marker M indicating the position of the electronic cassette 11 is superimposed on the patient H is displayed on the operation panel 29 or the like as the detection result, in a case in which the position deviation between the electronic cassette 11 and the marker M is large in the composite image 88, the appearance of the marker M is also not good. Therefore, since it is considered that the position deviation of the detection result of the first position detection mechanism is smaller than the position deviation of the detection result of the second position detection mechanism, it is also effective from the viewpoint of the appearance of the marker M to use the detection result of the first position detection mechanism as much as possible in a case in which the detection result of the first position detection mechanism can be used.

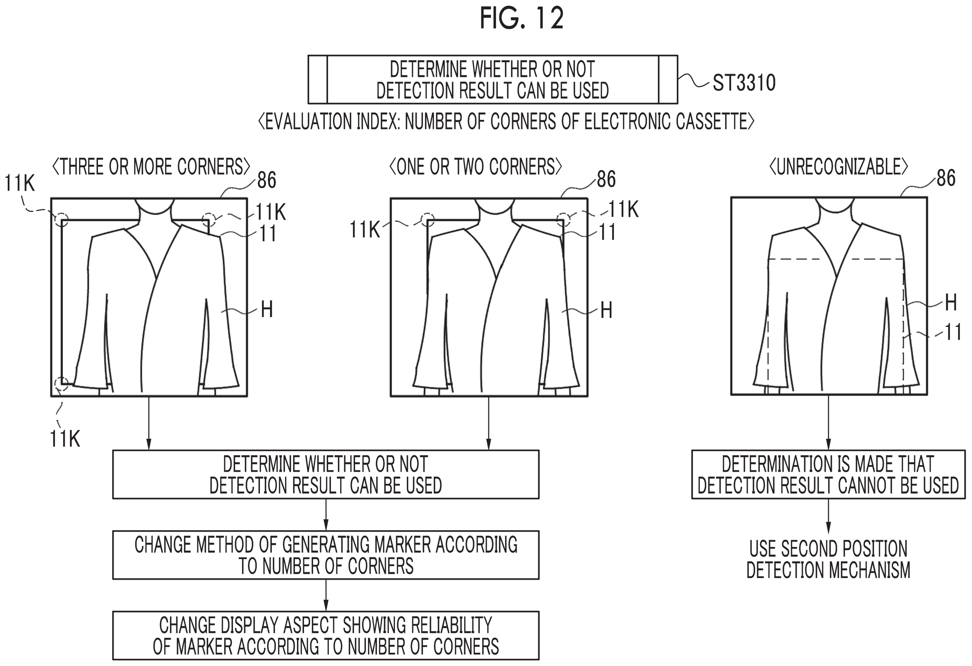

Furthermore, the content of the determination of whether to use step ST3310, which is a main difference from the first embodiment, will be specifically described with reference to FIG. 12. The processor 76 determines whether or not the detection result of the first position detection mechanism can be used based on the recognition result of the image recognition processing of step ST3300. As a result, the reliability degree of the detection result of the first position detection mechanism can be appropriately evaluated.

As an example, the recognition result includes the number of corners 11K of the electronic cassette 11 as the item to be recognized. Depending on the size and the position of the electronic cassette 11, the entire electronic cassette 11 may be hidden in the shadow of the patient H as shown in FIG. 7, or a part of the four corners 11K may be hidden. The processor 76 evaluates the reliability degree of the detection result of the first position detection mechanism by using the number of corners 11K of the electronic cassette 11 as the evaluation index, and determines whether or not the detection result of the first position detection mechanism can be used.

As shown in FIG. 12, in a case in which one or more corners 11K are recognized, the processor 76 uses the detection result of the first position detection mechanism. On the other hand, in a case in which no corner 11K is recognized (indicated by "unrecognizable" in FIG. 12), the processor 76 determines that the detection result of the first position detection mechanism cannot be used, and uses the detection result of the second position detection mechanism. By performing such a determination, the determination as to whether the detection result can be used can be simplified.

In addition, in a case in which the detection result of the first position detection mechanism is used, in a case in which the number of the recognized corners 11K is three or more, the processor 76 can derive the position of the center of the detection surface of the electronic cassette 11 and the cassette position including the contour of the electronic cassette 11 from the positions of the three or more corners 11K. As a result, it is possible to generate the marker M indicating the contour. However, in a case in which the number of the recognized corners 11K is one or two, the processor 76 cannot specify the position of the center of the detection surface of the electronic cassette 11 and the contour based only on the position of the corner 11K. In this case, the processor 76 generates the marker M based on the positions of one or two corners 11K and the preset size information of the electronic cassette 11. As described above, the processor 76 changes the method of generating the marker M according to the number of the recognized corners. As a result, the marker M can be generated by an appropriate method according to the recognition result. Such processing of generating the marker M is performed, of course, in a case of generating the composite image 88 of step ST3700 shown in FIG. 11.

In addition, in this case, the processor 76 may display, on the operation panel 29, the composite image 88 in which the marker M indicating the position of the electronic cassette 11 and the patient H are superimposed on each other as the detection result, and may change the display aspect of the marker M in the composite image 88 according to the reliability degree. This is because the reliability degree of the detection result including the cassette position may change depending on the number of corners 11K. For example, the processor 76 changes the display aspect of the marker M generated from the positions of the three or more corners 11K and the display aspect of the marker M generated from the positions of the one or two corners 11K. As a result, the operator OP can ascertain the high or low reliability degree from the marker M. As the display aspect of the marker M, any aspect may be used as long as the two can be identified as described in FIG. 10. For example, the thickness of the line of the marker M, the color or brightness of the line, the line type, and the like are changed. In addition, it is preferable to make the marker M having high reliability degree conspicuous because it is easy to intuitively ascertain that the reliability degree is high.

### Modification Example 1 of Second Embodiment

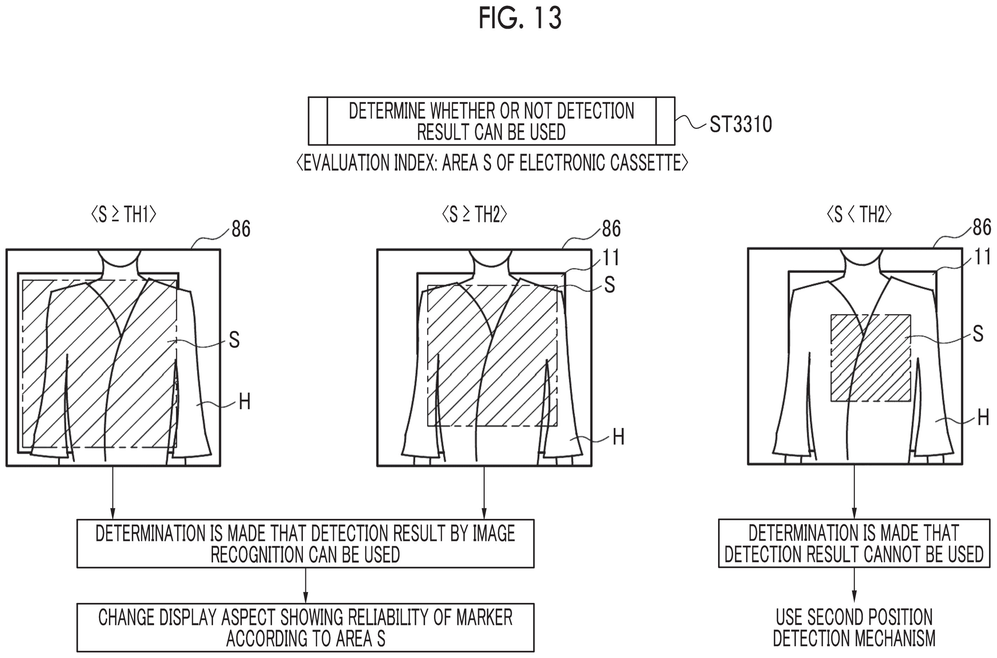

In addition, the evaluation index for the reliability degree of the detection result by the image recognition is not limited to the number of corners 11K of the electronic cassette 11. For example, as shown in FIG. 13, an area S recognized as the region of the electronic cassette 11 may be used as the evaluation index. Here, the area S is the area of the region of the electronic cassette 11 recognized by the machine learning model 87 based on the camera image 86, and does not necessarily match the position or the size of the electronic cassette 11 actually shown in the camera image 86. The machine learning model 87, for example, estimates a position of the contour of the electronic cassette 11 from a region of a part of the electronic cassette 11 appearing in the camera image 86, and outputs an area of the entire region within the contour as the area S. The processor 76 determines that the reliability degree of the detection result by the image recognition is high in a case in which the area S is close to the size of the electronic cassette 11 by comparing the area S with the actual size of the electronic cassette 11. In the example shown in FIG. 13, the processor 76 uses two threshold values of a threshold value TH1 and a threshold value TH2. The threshold value TH1 is larger than the threshold value TH2. Then, in a case in which the area S is equal to or larger than the threshold value TH2, the processor 76 determines that the detection result by the image recognition, that is, the detection result of the first position detection mechanism can be used. On the other hand, in a case in which the area S is smaller than the threshold value TH2, the processor 76 determines that the detection result of the first position detection mechanism cannot be used, and uses the detection result of the second position detection mechanism.

In addition, in a case in which the area S is equal to or larger than the threshold value TH2, the processor 76 changes the display aspect of the marker M according to whether or not the area S is equal to or larger than the threshold value TH1, as in the example shown in FIG. 12. This is because it is considered that the reliability degree changes depending on whether or not the area S is equal to or larger than the threshold value TH1. As a result, the operator OP can ascertain the reliability degree of the marker M through the display aspect of the marker M.

### Modification Example 2 of Second Embodiment

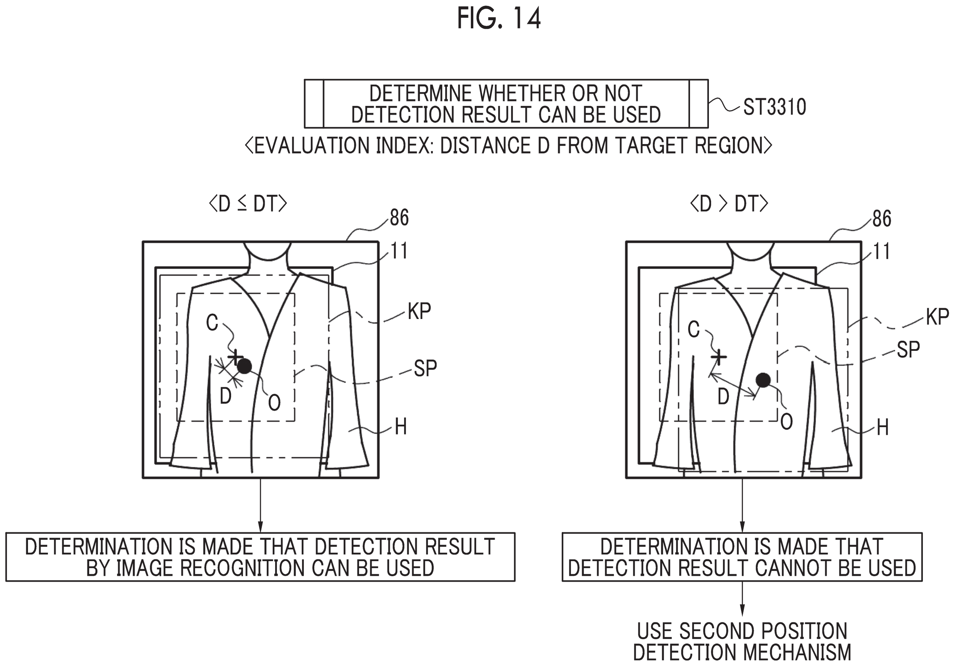

In addition, as shown in FIG. 14, a distance D from the target region may be used as the evaluation index. Here, the distance D is a distance between a center O of a region KP of the electronic cassette 11 recognized by the machine learning model 87 based on the camera image 86 and a center C of an irradiation field SP of the radiation source 15. The operator OP can perform rough alignment of the relative positional relationship between the irradiation field SP of the radiation source 15 and the electronic cassette 11 by visual observation. Therefore, the irradiation field SP set by the operator OP can be used as the target region, and the distance D representing how far the recognized region KP of the electronic cassette 11 is from the target region can be used as the evaluation index for the reliability degree of the detection result by the image recognition. That is, the idea is that, in a case in which the region KP of the electronic cassette 11 is recognized near the position roughly aligned by the operator OP, the recognition result can be estimated to have high reliability degree.

The processor 76 determines that the detection result by the image recognition can be used in a case in which the distance D is equal to or less than a threshold value DT by using the threshold value DT. On the other hand, in a case in which the distance D is larger than the threshold value DT, the processor 76 determines that the detection result by the image recognition cannot be used. The threshold value DT is, for example, 10 mm. There is a standard that an allowable range of the error of the irradiation field SP is within 1% in a case in which a source-to-image distance (SID) is 1000 mm. The numerical example of the threshold value DT of 10 mm is based on this standard.

### Modification Example 3 of Second Embodiment

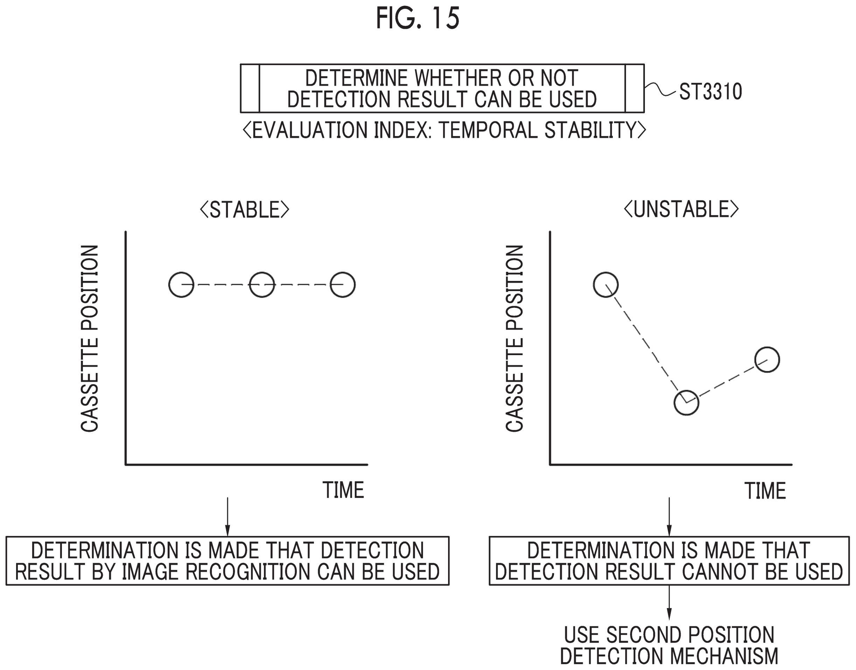

In addition, as shown in FIG. 15, temporal stability of the recognition result may be used as the evaluation index. Here, the temporal stability is an index indicating whether or not the cassette position of the electronic cassette 11 recognized by the machine learning model 87 based on the camera image 86 is stably detected over time. That is, in a case in which the machine learning model 87 repeats the detection of the cassette position by the image recognition a plurality of times, as shown in the graph on the left side of FIG. 15, in a case in which the detected cassette position is at substantially the same position, the temporal stability is high, and a determination is made that the reliability degree of the recognition result is high. In this case, the detection result by the image recognition, that is, the detection result of the first position detection mechanism is used. On the contrary, as shown in the graph on the right side of FIG. 15, in a case in which the fluctuation of the cassette position output a plurality of times is large, the temporal stability is low, and a determination is made that the reliability degree of the recognition result is low. In this case, the detection result of the second position detection mechanism is used.

As described above, various evaluation indices shown in FIGS. 12 to 15 can be considered as the evaluation index. Of course, other evaluation indices may be used.

### Third Embodiment

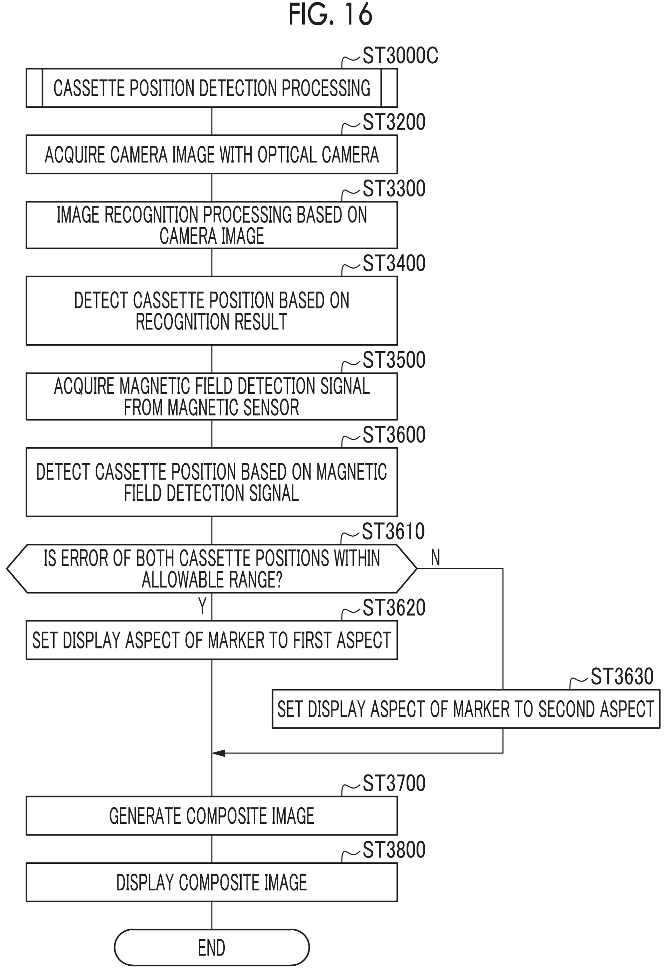

In addition, in the first embodiment and the second embodiment, a single mode in which in which the detection results of the first position detection mechanism and the second position detection mechanism are selectively used has been described as an example, but as in the third embodiment shown in FIG. 16, a combined mode in which the detection results of both the first position detection mechanism and the second position detection mechanism are used may be provided. Since the single mode and the combined mode are provided, it is possible to use the radiography system according to the situation as compared with a case in which only the single mode is provided.

A way of using the combined mode is as follows as an example. For example, in the combined mode, the processor 76 uses the detection result of the second position detection mechanism to determine a reliability degree of the detection result of the first position detection mechanism. As a result, it is possible to verify the reliability degree of the detection result of the first position detection mechanism, and it is possible to present a more accurate detection result in some cases. The configuration of the radiography system 2 of the third embodiment is substantially the same as that of the first and second embodiments. Hereinafter, the same configurations will not be described, and the differences will be mainly described.

FIG. 16 is a flowchart showing a processing procedure of the cassette position detection processing in the third embodiment. Step ST3000C shown in FIG. 16 is an example of step ST3000 shown in FIG. 8 of the first embodiment. In the first embodiment, step ST3000A shown in FIG. 9 is adopted, but in the third embodiment, step ST3000C is adopted as step ST3000.

In step ST3000C shown in FIG. 16, the same step numbers are assigned to steps having the same contents as the steps included in step ST3000A shown in FIG. 9. The processing having the same step number will be omitted from the description in principle. In step ST3000C, the processor 76 first operates the first position detection mechanism, acquires the camera image 86 in step ST3200, and executes the image recognition processing based on the camera image 86 of step ST3300. In step ST3400, the processor 76 detects the cassette position based on the recognition result of the image recognition processing.

The third embodiment is characterized in that the combined mode is used, so that the processor 76 operates the second position detection mechanism after step ST3400, executes steps ST3500 and ST3600, and detects the cassette position based on the magnetic field detection signal.

Then, in step ST3610, the processor 76 determines whether or not the error of the cassette position of both the cassette position by the first position detection mechanism and the cassette position by the second position detection mechanism is within a preset allowable range. In a case in which the error is within the allowable range (Y in step ST3610), the processor 76 proceeds to step ST3620. In step ST3620, the processor 76 determines that the reliability degree of the detection result of the first position detection mechanism is high, and sets the display aspect of the marker M to a first aspect. On the other hand, in a case in which the error is outside the allowable range (N in step ST3610), the processor 76 proceeds to step ST3630. In step ST3630, the processor 76 determines that the reliability degree of the detection result of the first position detection mechanism is low, and sets the display aspect of the marker M to a second aspect. Then, in step ST3700, the processor 76 generates the composite image 88 including the marker M based on the detection result of the first position detection mechanism, and applies the first aspect or the second aspect to the display aspect of the marker M. As a result, the operator OP can ascertain the reliability degree of the detection result.

The allowable range is, for example, 10 mm. The numerical example of 10 mm is based on the same reason as the threshold value DT.

In addition, as described above, basically, the reliability degree of the detection result of the first position detection mechanism is higher than the reliability degree of the detection result of the second position detection mechanism. However, since the first position detection mechanism employs an optical detection method, the reliability of the detection result of the first position detection mechanism may be reduced depending on the illumination state of the imaging environment, for example, in a situation in which the reflection of the illumination light occurs on the surface of the electronic cassette 11 and the contour of the electronic cassette 11 is difficult to visually recognize. In such a case, it can be recognized that the significance is in using the detection result of the second position detection mechanism that is not affected by the illumination light because of the non-optical detection method as a basis for determining the reliability degree of the detection result of the first position detection mechanism.

In addition, as in the above example, the detection result of the second position detection mechanism may be displayed simply as reference information instead of using the detection result of the second position detection mechanism to determine the reliability degree of the detection result of the first position detection mechanism. Even in a case in which the operator OP checks the detection result of the first position detection mechanism with priority, the detection result of the second position detection mechanism can also be used as reference information in a case in which the detection result of the second position detection mechanism is also displayed.

### Other

In the first position detection mechanism, the cassette position of the electronic cassette 11 is detected by the image recognition based on the camera image 86. According to the image recognition, it is possible to detect the posture of the electronic cassette 11 in addition to the cassette position. As described above, the processor 76 may detect the posture of the electronic cassette 11 based on the camera image 86.

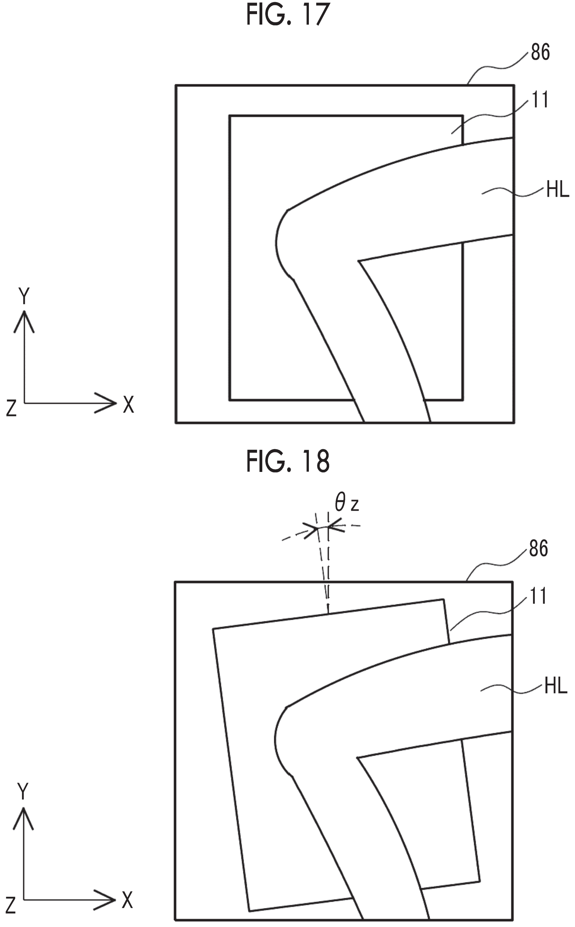

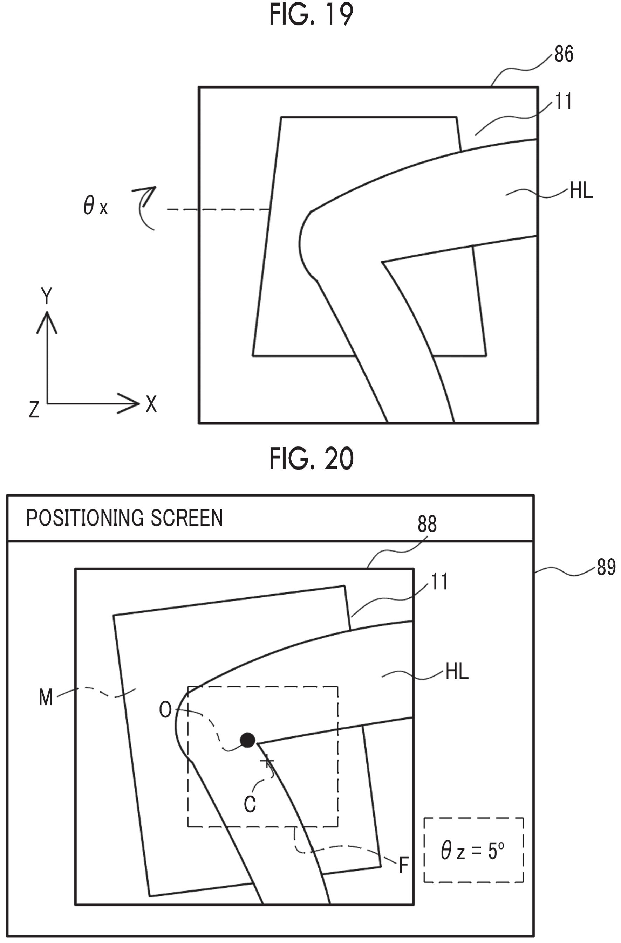

FIGS. 17 to 19 show a state in which the subject is a foot HL of the patient and the electronic cassette 11 is placed on a joint of the foot HL to perform imaging. FIG. 17 shows the electronic cassette 11 in the reference posture, and in a case in which this posture is set as the reference posture, FIG. 18 shows a posture in which the electronic cassette 11 is rotated by an angle θz about the Z axis. As described above, in a case in which the electronic cassette 11 is rotated about the Z axis from the reference posture, the electronic cassette 11 appear in the camera image 86 in a form in which the four sides are inclined by the same angle. In addition, FIG. 19 shows a posture in which the electronic cassette 11 is rotated by an angle θx about the X axis. As described above, in a case in which the electronic cassette 11 is rotated about the X axis from the reference posture, the electronic cassette 11 appears in the camera image 86 in a trapezoidal form, in which the upper side of the electronic cassette 11 is shorter than the lower side, and the two sides on both sides are inclined. As described above, in a case in which the posture of the electronic cassette 11 is changed, the form of the electronic cassette 11 appearing in the camera image 86 is changed, so that the posture of the electronic cassette 11 can be detected from the camera image 86.

For example, as shown in FIG. 20, in a case in which the posture of the electronic cassette 11 is detected, the processor 76 displays the rotation angle of the electronic cassette 11 on the positioning screen 89. In FIG. 20, there is an angle display of "θz = 5°", which indicates that the rotation is 5° about the Z axis. The operator OP can ascertain the posture of the electronic cassette 11 by the angle display.

Since the posture of the electronic cassette 11 is useful information in the positioning, the usability is further improved.

In addition, in the above-described embodiment, as an example in which the detection result of the first position detection mechanism is preferentially used, an example in which determination as to whether the detection result can be used is made by image recognition has been described. In addition to this example, as an example in which the detection result of the first position detection mechanism is preferentially used, for example, in a case of manual selection, the content may be that the default setting is set as the first position detection mechanism, and the first position detection mechanism is used unless the second position detection mechanism is selected.

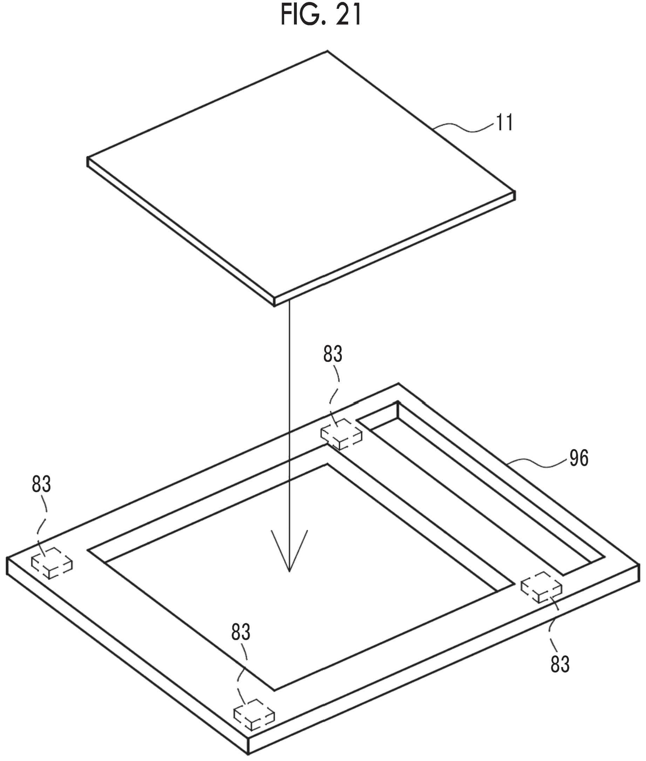

In each of the above-described embodiments, the example has been described in which the magnetic field generator 83 is provided in the electronic cassette 11, but the concept of providing the magnetic field generator 83 in the electronic cassette 11 also includes a case in which the magnetic field generator 83 is provided in a holder 96 that is an accessory of the electronic cassette 11 as shown in FIG. 21. The holder 96 can be attached to the electronic cassette 11, and can be used, for example, in a state of being attached to the electronic cassette 11. A handle is provided in the holder 96, and the handleability of the electronic cassette 11 is improved by attaching the holder 96 to the electronic cassette 11. As described above, the holder 96 is an accessory that is used in combination with the electronic cassette 11, and the four corners of the holder 96 correspond to the four corners of the electronic cassette 11. Therefore, by providing the magnetic field generator 83 at the four corners of the holder 96, it is possible to detect the cassette position of the electronic cassette 11.

In addition, it is preferable that the processor 76 stops transmission and reception of the non-optical signal (magnetic field or radio wave) in the second position detection mechanism at a timing of the radiography. This is because there is a concern that the magnetic field or the radio wave may adversely affect the image quality of the radiation image 20.

In addition, the above-described embodiment discloses the following technology.

### Supplementary Note 1

A radiography system for imaging a subject, including:
a radiation generation apparatus;
an electronic cassette;
a first position detection mechanism that includes an optical camera that is provided in the radiation generation apparatus and capable of imaging the electronic cassette, and detects a position of the electronic cassette based on a camera image captured by the optical camera;
a second position detection mechanism that includes a generator that generates a non-optical signal and a sensor that detects the non-optical signal, in which one of the generator and the sensor is provided in the radiation generation apparatus and the other is provided in the electronic cassette, and detects a position of the electronic cassette based on the non-optical signal; and
a processor that is configured to execute control of outputting a detection result of at least one of the first position detection mechanism or the second position detection mechanism.

### Supplementary Note 2

The radiography system according to Supplementary Note 1, further including:
two operation modes of a single mode in which the detection results of the first position detection mechanism and the second position detection mechanism are selectively used and a combined mode in which the detection results of both the first position detection mechanism and the second position detection mechanism are used.

### Supplementary Note 3

The radiography system according to Supplementary Note 1 or 2,
in which the processor is configured to:
display, on a display, a composite image in which a marker indicating the position of the electronic cassette and the subject are superimposed on each other as the detection result; and
change a display aspect of the marker in the composite image according to whether the detection result is acquired from the first position detection mechanism or the second position detection mechanism.

### Supplementary Note 4

The radiography system according to any one of Supplementary Notes 1 to 3,
in which the processor is configured to determine a way of using the first position detection mechanism and the second position detection mechanism based on a preset condition.

### Supplementary Note 5

The radiography system according to Supplementary Note 4,
in which the processor is configured to preferentially use the detection result of the first position detection mechanism.

### Supplementary Note 6

The radiography system according to Supplementary Note 5,
in which the processor is configured to:
determine whether or not the detection result of the first position detection mechanism is usable; and
use the detection result of the second position detection mechanism in a case in which a determination is made that the detection result of the first position detection mechanism is unusable.

### Supplementary Note 7

The radiography system according to Supplementary Note 6,
in which the processor is configured to:
execute image recognition processing of performing image recognition of the electronic cassette from the camera image; and
determine, based on a recognition result of the image recognition processing, whether or not the detection result of the first position detection mechanism is usable.

### Supplementary Note 8

The radiography system according to Supplementary Note 7,
in which the recognition result includes at least one item among the number of corners of the electronic cassette that are recognized, an area of a region recognized as a part of the electronic cassette, a position of the region recognized as the part of the electronic cassette, or temporal stability of the recognition result, and
the processor is configured to evaluate a reliability of the detection result of the first position detection mechanism by using the at least one item as an evaluation index, and determine whether or not the detection result of the first position detection mechanism is usable.

### Supplementary Note 9

The radiography system according to Supplementary Note 8,
in which the evaluation index is the number of corners of the electronic cassette that are recognized, and
the processor is configured to:
   use the detection result of the first position detection mechanism in a case in which one or more corners are recognized; and
   use the detection result of the second position detection mechanism in a case in which no corner is recognized.

### Supplementary Note 10

The radiography system according to Supplementary Note 8 or 9,
in which the processor is configured to:
display, on a display, a composite image in which a marker indicating the position of the electronic cassette and the subject are superimposed on each other as the detection result;
generate the marker based on positions of three or more recognized corners in a case in which the number of the recognized corners is three or more; and
generate the marker based on positions of one or two recognized corners and preset size information of the electronic cassette in a case in which the number of the recognized corners is one or two.

### Supplementary Note 11

The radiography system according to any one of Supplementary Notes 8 to 10,
in which the processor is configured to:
display, on a display, a composite image in which a marker indicating the position of the electronic cassette and the subject are superimposed on each other as the detection result; and
change a display aspect of the marker in the composite image according to the reliability degree.

### Supplementary Note 12

The radiography system according to any one of Supplementary Notes 2 to 11,
in which the processor is configured to, in the combined mode, use the detection result of the second position detection mechanism to determine a reliability degree of the detection result of the first position detection mechanism.

### Supplementary Note 13

The radiography system according to Supplementary Note 12,
in which the processor is configured to:
display, on a display, a composite image in which a marker indicating the position of the electronic cassette and the subject are superimposed on each other as the detection result; and
change a display aspect of the marker in the composite image according to the reliability degree.

### Supplementary Note 14

The radiography system according to any one of Supplementary Notes 1 to 13,
in which the processor is configured to detect a posture of the electronic cassette based on the camera image.

### Supplementary Note 15

The radiography system according to any one of Supplementary Notes 1 to 14,
in which the non-optical signal is a magnetic field or a radio wave, and
the sensor is a magnetic sensor or a wireless receiver.

### Supplementary Note 16

The radiography system according to any one of Supplementary Notes 1 to 15,
in which the processor is configured to stop transmission and reception of the non-optical signal in the second position detection mechanism at a timing of the radiography.

In the above-described embodiment, the processing executed by the processor 76 of the radiography system 2 is executed by any computer. In addition, any computer may execute these processes by a processor as hardware, a program as software, or a combination thereof. In such a case, the processor is configured to execute various types of processing in the present embodiment in cooperation with the program, and may function as each unit or each means in the present embodiment. In addition, the execution order of the processing by the processor is not limited to the order described above and may be changed as appropriate.

Any computer may be a general-purpose computer, a computer for a specific use, a workstation, or another system capable of executing each process. The processor may be configured by one or a plurality of hardware, and the type of hardware is not limited. For example, the processor may be composed of hardware such as a central processing unit (CPU), a micro processing unit (MPU), a programmable logic device such as a field programmable gate array (FPGA), a dedicated circuit for executing specific processing, such as an application specific integrated circuit (ASIC), a graphic processing unit (GPU), or a neural processing unit (NPU). In addition, the types of hardware may be a combination of different types of hardware. In a case where a plurality of hardware are configured to execute one or a plurality of processes of a certain processor, the plurality of hardware may be present in devices physically separated from each other, or may be present in the same device. In addition, in any of the embodiments, the order of each processing by the processor is not limited to the above order, and may be changed as appropriate. The hardware is composed of an electric circuit (circuitry) in which circuit elements such as semiconductor elements are combined.

Furthermore, the program may be software such as firmware or a microcode. In addition, the program may be, for example, a program module group, and each function thereof may be realized by a processor configured to execute each function. The program may be a program code or a plurality of code segments stored in one or a plurality of non-transitory computer-readable media (for example, a storage medium or other storage). The program may be stored in a plurality of non-transitory computer-readable media existing in devices physically separated from each other. The program code or code segment may represent any combination of a procedure, a function, a subprogram, a routine, a subroutine, a module, a software package, a class, or an instruction, a data structure, or a program statement. The program code or code segment may be connected to another code segment or a hardware circuit by transmitting and receiving information, data, an argument, a parameter, or a content of a memory.

The technology of the present disclosure can also be combined with various embodiments and/or various modification examples described above, as appropriate. In addition, the present disclosure is not limited to the above-described embodiments, and various configurations can be adopted without departing from the gist of the present disclosure. Further, the technology of the present disclosure includes a storage medium that stores the program in a non-transitory manner, in addition to the program. The storage medium is, for example, a non-transitory computer-readable storage medium such as a universal serial bus (USB) memory, a flexible disk, or a compact disc read only memory (CD-ROM). The program may be provided online through a network such as the Internet. The disclosed technology also applies to a program product in addition to the program. The program product includes products in all aspects for providing a program. Like the program, the program product may be provided by being stored in a non-transitory computer-readable storage medium or may be provided online.

The above descriptions and illustrations are detailed descriptions of portions related to the technology of the present disclosure and are merely examples of the technology of the present disclosure. For example, the above description of the configuration, the function, the operation, and the effect are the description of examples of the configuration, the function, the operation, and the effect of the parts according to the technology of the present disclosure. Accordingly, it goes without saying that unnecessary parts may be deleted, new elements may be added, or replacements may be made with respect to the above-described contents and the above-shown contents within a range that does not deviate from the gist of the technology of the present disclosure. In addition, in the above descriptions and illustrations, the description of, for example, common technical knowledge that does not need to be particularly described to enable the implementation of the technology of the present disclosure is omitted in order to avoid confusion and facilitate the understanding of portions related to the technology of the present disclosure.

In the present specification, "A and/or B" is synonymous with "at least one of A or B". That is, "A and/or B" means that it may be only A, only B, or a combination of A and B. Further, in the specification, the same concept as "A and/or B" is applied to a case where the connection of three or more matters is expressed by "and/or".

All documents, patent applications, and technical standards described in the present specification are incorporated by reference into the present specification to the same extent as in a case where the individual documents, patent applications, and technical standards were specifically and individually indicated to be incorporated by reference.

Explanation of References

## Claims

1. A radiography system for imaging a subject, comprising:
a radiation generation apparatus;
an electronic cassette;
a first position detection mechanism that includes an optical camera that is provided in the radiation generation apparatus and capable of imaging the electronic cassette, and detects a position of the electronic cassette based on a camera image captured by the optical camera;
a second position detection mechanism that includes a generator that generates a non-optical signal and a sensor that detects the non-optical signal, in which one of the generator and the sensor is provided in the radiation generation apparatus and the other is provided in the electronic cassette, and detects a position of the electronic cassette based on the non-optical signal; and
a processor that is configured to execute control of outputting a detection result of at least one of the first position detection mechanism or the second position detection mechanism.

2. The radiography system according to claim 1, further comprising:
two operation modes of a single mode in which the detection results of the first position detection mechanism and the second position detection mechanism are selectively used and a combined mode in which the detection results of both the first position detection mechanism and the second position detection mechanism are used.

3. The radiography system according to claim 1 or 2,
wherein the processor is configured to:
display, on a display, a composite image in which a marker indicating the position of the electronic cassette and the subject are superimposed on each other as the detection result; and
change a display aspect of the marker in the composite image according to whether the detection result is acquired from the first position detection mechanism or the second position detection mechanism.

4. The radiography system according to any one of the preceding claims,
wherein the processor is configured to determine a way of using the first position detection mechanism and the second position detection mechanism based on a preset condition.

5. The radiography system according to claim 4,
wherein the processor is configured to preferentially use the detection result of the first position detection mechanism.

6. The radiography system according to claim 5,
wherein the processor is configured to:
determine whether or not the detection result of the first position detection mechanism is usable; and
use the detection result of the second position detection mechanism in a case in which a determination is made that the detection result of the first position detection mechanism is unusable.

7. The radiography system according to claim 6,
wherein the processor is configured to:
execute image recognition processing of performing image recognition of the electronic cassette from the camera image; and
determine, based on a recognition result of the image recognition processing, whether or not the detection result of the first position detection mechanism is usable.

8. The radiography system according to claim 7,
wherein the recognition result includes at least one item among the number of corners of the electronic cassette that are recognized, an area of a region recognized as a part of the electronic cassette, a position of the region recognized as the part of the electronic cassette, or temporal stability of the recognition result, and
the processor is configured to evaluate a reliability of the detection result of the first position detection mechanism by using the at least one item as an evaluation index, and determine whether or not the detection result of the first position detection mechanism is usable.

9. The radiography system according to claim 8,
wherein the evaluation index is the number of corners of the electronic cassette that are recognized, and
the processor is configured to:
use the detection result of the first position detection mechanism in a case in which one or more corners are recognized; and
use the detection result of the second position detection mechanism in a case in which no corner is recognized.

10. The radiography system according to claim 8 or 9,
wherein the processor is configured to:
display, on a display, a composite image in which a marker indicating the position of the electronic cassette and the subject are superimposed on each other as the detection result;
generate the marker based on positions of three or more recognized corners in a case in which the number of the recognized corners is three or more; and
generate the marker based on positions of one or two recognized corners and preset size information of the electronic cassette in a case in which the number of the recognized corners is one or two.

11. The radiography system according to any one of claims 8 to 10,
wherein the processor is configured to:
display, on a display, a composite image in which a marker indicating the position of the electronic cassette and the subject are superimposed on each other as the detection result; and
change a display aspect of the marker in the composite image according to the reliability degree.

12. The radiography system according to any one of claims 2 to 11,
wherein the processor is configured to, in the combined mode, use the detection result of the second position detection mechanism to determine a reliability degree of the detection result of the first position detection mechanism.

13. The radiography system according to claim 12,
wherein the processor is configured to:
display, on a display, a composite image in which a marker indicating the position of the electronic cassette and the subject are superimposed on each other as the detection result; and
change a display aspect of the marker in the composite image according to the reliability degree.

14. The radiography system according to any one of the preceding claims,
wherein the processor is configured to detect a posture of the electronic cassette based on the camera image.

15. The radiography system according to any one of the preceding claims,
wherein the non-optical signal is a magnetic field or a radio wave, and
the sensor is a magnetic sensor or a wireless receiver.

16. The radiography system according to any one of the preceding claims,
wherein the processor is configured to stop transmission and reception of the non-optical signal in the second position detection mechanism at a timing of the radiography.
